# EUROPEAN PATENT SPECIFICATION

(11) **EP 2 619 323 B1**
(45) Date of publication and mention of the grant of the patent: **08.11.2017**
(21) Application number: 11767205.5
(22) Date of filing: 21.09.2011
(51) Int. Cl.: C12Q 1/68, B01L 3/00, G01N 27/447, B01L 9/00

(54) **METHODS AND APPARATUS FOR AMPLIFYING NUCLEIC ACIDS**
VERFAHREN UND VORRICHTUNG ZUR NUKLEINSÄUREAMPLIFIKATION
PROCÉDÉS ET APPAREIL POUR L'AMPLIFICATION D'ACIDES NUCLÉIQUES

(30) Priority: 21.09.2010 GB 201015803
(43) Date of publication of application: 31.07.2013
(73) Proprietor: The University of Hull, Kingston-upon-Hull HU6 7RX (GB)
(72) Inventor: HASWELL, Stephen John, Cottingham HU16 4RL (GB); SHAW, Kirsty Jane, Hull HU6 7UD (GB); DOCKER, Peter, Hull HU5 3JS (GB)
(74) Representative: Hartley, Andrew Philip
(86) International application number: PCT/EP2011/066392
(87) International publication number: WO 2012/038462

(56) References cited:
- WO-A1-99/60397
- WO-A1-2009/016652
- WO-A1-2010/041088
- WO-A2-2009/112594
- US-A1- 2002 090 320
- US-A1- 2004 238 355
- US-A1- 2007 172 388
- JENNIFER A. OAKLEY ET AL: "Development of a bi-functional silica monolith for electro-osmotic pumping and DNA clean-up/extraction using gel-supported reagents in a microfluidic device", LAB ON A CHIP, vol. 9, no. 11, 1 January 2009 (2009-01-01), page 1596, XP55012895, ISSN: 1473-0197, DOI: 10.1039/b820553a

## Description

The invention relates to methods for amplifying nucleic acids.

WO 2010/041088 describes a microfluidic device and a method of operating the microfluidic device. The microfluidic device has a reaction chamber in which a PCR reaction is performed. The reaction chamber is filled with a gel and the reagents necessary for performing the PCR reaction are held within the matrix of the gel until the microfluidic device is used. The microfluidic device can be stored with the reagents in a stable form prior to use. The microfluidic device is also suitable for automated use. The microfluidic device is provided with a separation chamber for separation of DNA to be amplified from other cell components.

According to the invention, there is provided, a method of amplifying nucleic acid as defined in claim 1.

Preferably the method also includes analysing nucleic acid products of the PCR amplification within the microfluidic device. For example, the nucleic acid products may be analysed by performing electrophoretic separation of the PCR products.

The sample may be a buccal swab sample or a blood sample.

The gel medium may contain at least one of nucleoside triphosphates, primer nucleic acid and polymerase enzyme within the gel matrix. Preferably all of these reagents are supported within the matrix of the gel medium.

The gel medium preferably has a homogenous composition. In addition the PCR reagents within the matrix of the gel medium are preferably homogenously distributed within the gel medium.

The following is a more detailed description of embodiments of the invention, by way of example, reference being made to the appended schematic drawings in which:
Figure 1 is a plan view from above of a microfluidic device;
Figure 2 is a cross-sectional view of the microfluidic device of Figure 1, taken on the line A-A in Figure 1; and
Figure 3 is a plan view from above showing the microfluidic device of Figures 1 and 2 while the microfluidic device is held by detection apparatus.

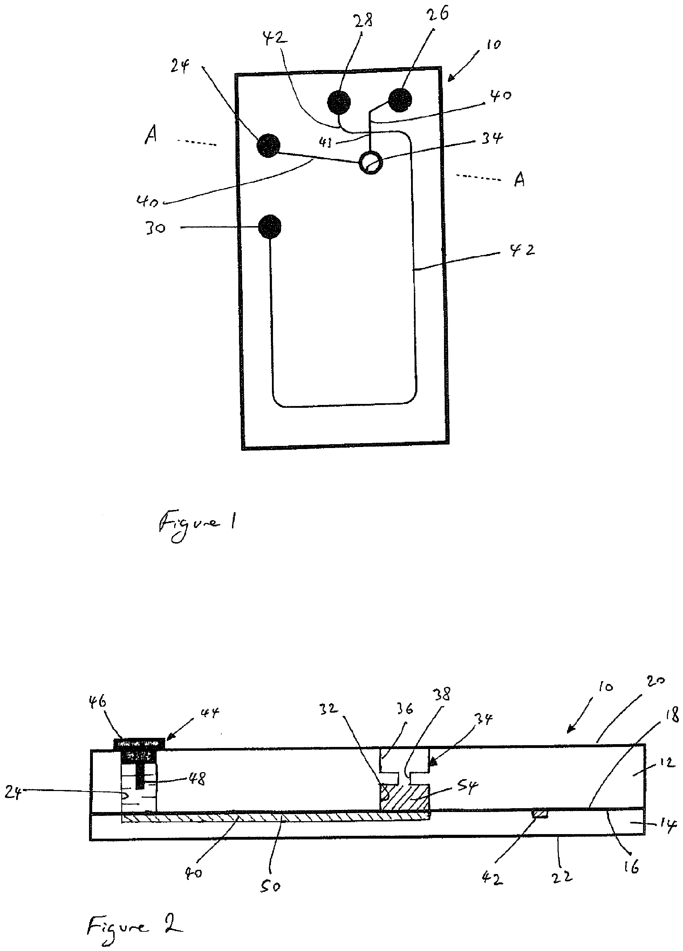

As seen in Figure 1, the microfluidic device 10 is rectangular and may, by way of example, have a length of about 120mm and a width of about 60mm. Referring to Figure 2, the microfluidic device 10 is formed from an upper rectangular glass plate 12 and a lower rectangular glass plate 14. The lower surface 16 of the upper glass plate 12 is bonded to the upper surface 18 of the lower glass plate 14 by a suitable known method. Thermal bonding is the preferred method. The upper surface 20 of the upper plate 12 forms an upper external surface of the microfluidic device 10 and the lower surface 22 of the lower glass plate 14 forms a lower external surface of the microfluidic device 10.

As seen in Figure 2, the thickness (height as shown in Figure 2) of the upper glass plate 12 is greater than the thickness of the lower glass plate 14. By way of example, the upper glass plate may have a thickness of 3mm and the lower glass plate may have a thickness of 1mm.

The microfluidic device 10 is provided with first, second, third and fourth wells 24, 26, 28, 30. Each one of the wells 24, 26, 28, 30 serves to receive a respective electrode as will be discussed in more detail below. The first well 24 is shown in longitudinal (vertical) section in Figure 2. As best seen in Figure 2, each well takes the form of the cylindrical hole that has been drilled through the upper plate 12 between the upper and lower surfaces 20, 16 of the upper plate 12. Each one of the first, second, third and fourth wells, 24, 26, 28, 30 may have a diameter of about 2 to 3 mm.

The microfluidic device 10 also has an internal chamber 32 in which a polymerase chain reaction (PCR) amplification process is performed, as will be discussed below in more detail. The PCR chamber 32 is cylindrical in shape and has been formed by drilling a cylindrical well part of the way into the upper glass plate 12, starting from the lower surface 16 of the upper glass plate 12, before binding together of the upper glass plate 12 and the lower glass plate 14.

The microfluidic device 10 also has a sample collection well 34. The sample collection well 34 has an upper cylindrical portion 36 with a greater diameter and a lower cylindrical portion 38 with a lesser diameter. The lower portion 38 of the sample collection well 34 extends between the upper portion 36 and the PCR chamber 32, as shown in Figure 2. The sample collection well 34 is formed by firstly drilling into the upper glass plate 12 from the upper surface 20 with a larger diameter drill. This forms the upper portion 36. A smaller diameter drill is then used to drill between the upper portion 36 and the PCR chamber 32 so as to form the lower portion 38.

The microfluidic device 10 also has a sample transfer channel 40 and a separation channel 42.

As best seen in Figure 1, the sample transfer channel 40 extends from the first well 24 to the PCR chamber 32 and from the PCR chamber 32 to the second well 26. As seen in Figure 2, the sample transfer channel 40 opens directly into the first well 24 and also opens directly into the PCR chamber 32. Although not shown in the drawings, the sample transfer channel 40 opens directly into the second well 26. The cross-sectional dimensions of the sample transfer channel 40 will generally be less than 500 micrometers (although larger dimensions may be used). For example, the sample transfer channel 40 may have a width of about 100 micrometers and a depth of about 20 micrometers. The sample transfer channel 40 is formed by forming a groove of appropriate cross-section and dimensions in the upper surface 18 of the lower plate 14, before the upper plate 12 and the lower plate 14 are bonded together. On bonding of the two plates 12, 14, the lower surface 16 of the upper plate 12 closes the groove to form the sample transfer channel 40.

As seen in Figure 1, the separation channel 42 extends from the third well 28 to the fourth well 30. The separation channel 42 opens directly into both the third and fourth wells 28, 30. The separation channel 42 has similar dimensions, and is formed in a similar manner, to the sample transfer channel 40.

The sample transfer channel 40 and the separation channel 42 are in fluid communication with one another at an intersection 43 between the two channels 40, 42.

In addition, the microfluidic device 10 is provided with four electrode plugs 44, one of which is shown in Figure 2. The four electrode plugs 44 are preferably identical to one another, but need not be so. Each electrode plug 44 is formed from a cap 46 made of electrically non conductive material and an electrode 48 which passes through the cap 46. As shown in Figure 2, the cap 46 is sized so that it can be inserted into one of the wells 24, 26, 28, 30 so as to form a tight seal.

The surfaces of the first, second, third and fourth wells 24, 26, 28, 30, the PCR chamber 32, the sample collection well 34 and the sample transfer and separation channels 40, 42 are silanised to reduce binding of nucleic acid to the glass during use. Silanisation is performed after the upper and lower glass plates 12, 14 have been connected together. The microfluidic device 10 is cleaned with water and dried with air before being left to dry in an oven at 90°C overnight. The device 10 is then kept in a dessicator until silanisation is performed. (Similar measures are taken to treat the glassware used to hold the silanisation reagents.) Iso-octane (1000 microlitres) is mixed with trichloro (1H, 1H, 2H, 2H) perfluorooctyl silane (145 microlitres). The mixture is pumped into the microfluidic device 10 and moved through the channels 40, 42 and the wells/chambers 24, 26, 28, 30, 32, 34. After leaving for 5 minutes, the mixture is removed by pumping air through the microfluidic device. The microfluidic device 10 is then washed with iso-octane followed by drying with air. Finally, acetone, then air, then water are pumped through the microfluidic device 10. The microfluidic device 10 is dried in an oven for an hour before being filled with various gels as described below.

Firstly, the sample transfer channel 40 is filled with an agarose gel 50. In order to form the agarose gel 50, low melting point agarose is dissolved in nucleic acid free water and the solution is heated at 75°C for ten minutes. The final concentration of the agarose is 1.5% (weight:weight). The agarose gel 50 is inserted into the sample transfer channel 40 as follows. Firstly, the third and fourth wells 28, 30 and also the upper portion 36 of the sample collection well 34, are plugged with tight fitting plugs which occupy most of the space of the wells 28, 30, 36. Then, when the gel has formed, but whilst the gel is still in molten form, the gel is injected under pressure into the sample transfer channel 40 through the first well 24. During this process, the agarose gel 50 passes through the sample transfer channel 40 to the second well 26. In view of the fact that the sample collection well 34 is plugged, the agarose gel 50 does not enter, or enters only to a small degree, into the PCR chamber 32 (which is in fluid communication with the sample transfer channel 40). Once the agarose gel 50 reaches the base of the second well 26 injection is stopped. The gel is then allowed to solidify within the sample transfer channel 40 and the first and second wells 24, 26 are cleaned of gel.

The separation channel 42 is then filled with polyethylene oxide gel 52. The polyethylene oxide gel 52 is made by mixing polyethylene oxide to a concentration of 2.5% (weight:weight) in nucleic acid free Tris-EDTA buffer, by a prolonged stirring method. The gel 52 is introduced into the separation chamber 42 before it sets. In order to introduce the polyethylene oxide gel 52, the first and second wells 24, 26 and the sample collection well 34 are plugged. The molten gel is then introduced under pressure into the separation channel 42 via the third well 28. This process is continued until the polyethylene oxide gel reaches the base of the fourth well 30. After the polyethylene oxide gel 52 has set, gel is removed from the third and fourth wells 28, 30.

During the introduction of the polyethylene oxide gel 52 into the separation channel 42, a small amount of agarose gel 50 is dislodged from the intersection 43 of the sample transfer channel 40 and the separation channel 42. This portion of agarose gel 50 is carried to the fourth well 30 and does not serve any purpose.

In this example, the PCR chamber 32 is filled with a gel 54 which contains all the reagents necessary for performing PCR. The reagents are held within the matrix of the gel 54 within the PCR chamber 32. The reagents are the normal reagents used for performing PCR amplification, as is well known. Hence, the PCR reagents include nucleic acid sequences that act as primers in order to amplify predetermined portions of sample nucleic acid that is introduced into the PCR chamber 32. The reagents also include nucleoside triphosphates and a polymerase enzyme.

Preferably, the reagents will include a dye that binds to nucleic acid fragments that are produced in the PCR reaction. The dye may be, for example, a fluorescent dye or a dye having an intense absorption peak for colorimetric detection. The dye is used to aid detection of DNA fragments produced in the PCR reaction during subsequent separation of the DNA fragments, as discussed below.

By way of specific example, the PCR reagents may include the following components. The concentrations in the right hand column are the concentrations before dilution 1:1 with the gel, as described below.

| | | |
|---|---|---|
| **1.** autoclaved ultra-filtered water (pH 7.0) | 20.7µL | - |
| **2.** 10x PCR Buffer* | 2.5µL | 1x |
| **3.** dNTPs mix (25 mM each nucleotide) | 0.2µL | 200 µM (each nucleotide) |
| **4.** primer mix (25 pmoles/µL each primer) | 0.4µL | 0.4 µM (each primer) |
| **5.** Taq UNA polymerase (native enzyme) | 0.2µL | 1 Unit/25 µL |
| **6.** genomic DNA template (100 ng/µL) | 1.0µL | 100 ng/25 µL |

The gel containing the PCR reagents is formed as follows. Low melting point agarose is dissolved in nucleic acid free water at a concentration of 3% (weight:weight) and then heated at 75°C for ten minutes. When the gel has formed and whilst the gel is still in molten form, the gel is mixed with an equal volume of a solution containing all of the reagents that are required in the PCR chamber 32 (the reagents being present in the solution at double the final required concentration). After thorough mixing, the still molten gel, together with the PCR reagents, is injected through the lower portion 38 of the sample collection well 34 into the PCR chamber 32. The PCR gel 54 comes into contact with the agarose gel 50 contained in the sample transfer channel 40. The PCR gel 54 is then allowed to set in the PCR chamber 32.

As will be appreciated, the final concentration of the agarose in the PCR gel 54 is 1.5% (weight:weight).

The sample collection well 34, and the first, second, third and fourth wells 24, 26, 28, 30 remain empty.

Once the microfluidic device 10 has been loaded with the gels and reagents as discussed above, it can be kept refrigerated at 4°C for about four weeks before use.

The following is a description, by way of example, of one potential use of the microfluidic device 10. In this example, the microfluidic device 10 is used to amplify DNA contained in human cells obtained by way of buccal swab for the purposes of DNA profiling. The primers contained in the PCR gel 54 are chosen to amplify predetermined loci of human DNA used in DNA profiling. After amplification, the DNA fragments produced by the PCR reaction are analysed by electrophoretic separation.

Firstly, the first, second, third and fourth wells 24, 26, 28, 30 are filled with a suitable electrically conductive buffer. An electrode plug 44 is then inserted into each well 24, 26, 28, 30 so that the electrode 48 of the plug lies within the buffer and the cap 46 of the plug seals the well as shown in Figure 2. The electrodes 48 are used to apply voltages between the wells 24, 26, 28, 30 as discussed below.

A human cell sample is then taken by buccal swab. The sample bearing end of the swab is cut off and inserted into the sample collection well 34. Hence, there will be whole human cells on the end of the swab in the sample collection well 34. A suitable cell lysing solution is then introduced into the sample collection well 34. For example, the lysing solution may be a conventional guanidine lysing solution. The lysing solution lyses cells on the end of the swab and the lysing solution, together with the cell lysate migrates through the lower portion 38 of the sample collection well 34 into the PCR gel 54 in the PCR chamber 32. A cap may be inserted into the sample collection well 34, both to seal the well 34 and also to press the solution and cell lysate into the PCR gel 54.

During this process, the PCR gel 54 may act as a filter, preventing larger cell fragments from passing into the matrix of the PCR gel 54. However, this is not essential and will depend on the physical characteristics of the PCR gel 54.

Once the cell lysate has passed into the PCR gel 54, the PCR reaction may be commenced. As is well known, the PCR reaction involves cycling between two or three different temperatures. In the current method, the temperature within the PCR chamber 32 is cycled by a suitable known method. Suitable methods are described, for example, in WO2010/04108 The temperature cycling may be by way of a Peltier heater or by way of microwave heating, for example.

After the desired number of PCR temperature cycles have been completed, the desired loci of the human DNA will have been amplified. The PCR product DNA fragments are held within the PCR chamber 32.

A small amount of the PCR product DNA fragments is then loaded onto the polyethylene oxide gel 52 in the separation channel 42. In order to achieve this, the DNA fragments produced by the PCR reaction are moved to the intersection 43 between the sample transfer channel 40 and the separation channel 42 electrophoretically, by applying appropriate voltages to the electrodes 48 inserted into the first, second, third and fourth wells 24, 26, 28, 30.

For example, a positive voltage of 1,000v may be applied to the second well 26 for 15 seconds while the first, third and fourth wells 24, 28, 30 are held constant at 0v.

The DNA fragments are then electrophoretically separated in the polyethylene oxide gel 52 within the separation channel 42. In order to achieve this, a positive voltage of 8,500v is applied to the fourth well 30 for 25 minutes while the third well 28 is held at 0v, the second well 26 at 500V and the first well 24 at 1000v.

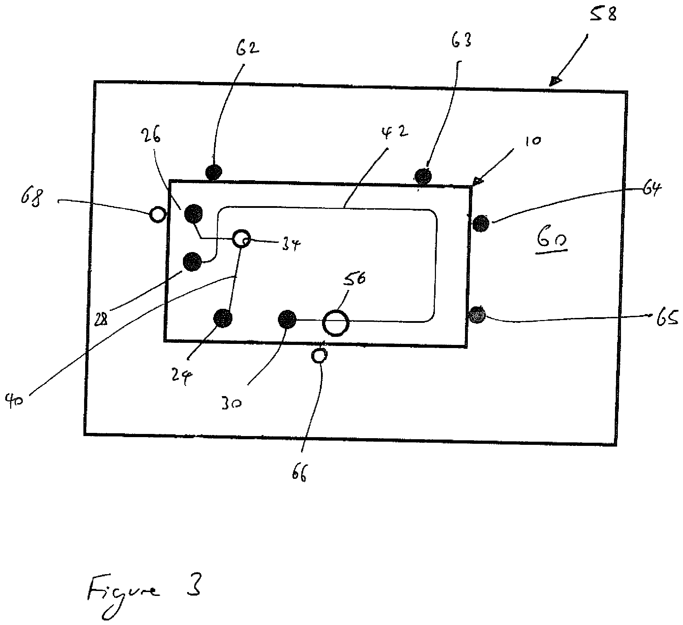

The DNA fragments move from the intersection 43 towards the fourth well 30. The DNA fragments can be detected by known methods, for example either by fluorimetry or colorimetry, as they pass a predefined point in the separation channel 42. For example, as shown in Figure 3, the DNA fragments may be detected as they pass through an analysis region 56 provided towards the an end of the separation channel 42 located adjacent the fourth well 30.

The method described above may be carried out readily in an automated manner, for example using the detection apparatus 58 shown in Figure 3. The detection apparatus 58 includes suitable electronics (not shown) for applying, in a known manner, the required voltages (as described above) to the electrodes 48. The detection apparatus 58 also includes a microcontroller (not shown) for controlling, in a known manner, the timing of the application of the required voltages to the electrodes 48. In addition, the detection apparatus 58 contains detection means, such as a fluorimeter or a colorimeter, for detecting the DNA fragments (bound to dye if applicable) as they pass the analysis region 56 of the microfluidic device 10.

As shown in Figure 3, the detection apparatus 58 has an upper surface 60 on which is placed the lower surface 22 of the microfluidic device 10.

In addition, the detection apparatus 58 has first, second, third and fourth fixed pegs 62, 63, 64, 65 and first and second moveable pegs 66, 68. The first moveable peg 66 is moveable towards and away from the first and second fixed pegs 62, 63 and the second moveable peg 68 is moveable towards and away from the third and fourth fixed pegs 64, 65. The first moveable peg 66 is spring loaded in the direction of the first and second fixed pegs 62, 63. The second moveable peg 68 is spring loaded in the direction of the third and fourth fixed pegs 64, 65. The pegs 62, 63, 64, 65, 66 and 68 are spaced so that the microfluidic device 10 can be located between the pegs as shown in Figure 3. The first moveable peg 66 urges the microfluidic device 10 towards the first and second fixed pegs 62, 63. The second moveable peg 68 urges the microfluidic device 10 towards the third and fourth fixed pegs 64, 65.

In this way, the microfluidic device 10 is held precisely in a predetermined position on the upper surface 60 of the detection apparatus 58. Only a very small degree of variation in the position of the microfluidic device 10 can occur.

The detection means (not shown) is positioned on the detection apparatus 58, so as to be aligned with the analysis region 56 of the microfluidic device 10 when the microfluidic device 10 is located between the pegs 62, 63, 64, 65, 66, 68 as described above.

The detection means emits a beam of light (or other electromagnetic radiation) through the analysis region 56 in order to detect the DNA fragments. The beam is wider than the width of the separation channel 42. In this way, the very small degree of variation in the position of the microfluidic device 10, when the microfluidic device 10 is located between the pegs 62, 63, 64, 65, 66, 68, will not influence detection of DNA fragments passing through the analysis region 56.

A number of advantages ensue from the microfluidic device 10 and the amplification method described above.

Firstly, the microfluidic device 10 and the method of operation do not require separation of nucleic acid from other cell components prior to DNA amplification in the PCR chamber 32 (although some separation may optionally occur if the matrix of the PCR gel 54 filters out larger cell fragments). Various known microfluidic devices used for DNA amplification use a distinct DNA separation step to separate DNA from other cellular components. The elimination of such a separation step simplifies both the design of the microfluidic device and also the amplification method, making the method more suitable for automated use by less skilled operators.

Secondly, in the microfluidic device 10 described above, the electrodes 48 are immersed in a conducting buffer which is in turn in contact with the gels 50 and 52 in the sample transfer and separation channels 40, 42. This is advantageous compared to inserting electrodes directly into the gels themselves - as it improves the electrical connection. When an electrode is inserted into a gel, the electrode is partially in contact with the gel itself (which is generally non-conductive) and partially in contact with a conducting liquid in the matrix of the gel. The overall electrical connection of such an arrangement may not be sufficient.

It will be appreciated that numerous changes may be made to the example given above without departing from the scope of the invention as defined in the claims.

Firstly, instead of adding a lysing solution into the sample collection well 34 as described above, the PCR gel 54 may contain a lysing agent. In this case whole cells enter into the PCR gel 54 and become lysed in the gel 54.

The sample need not be a buccal swab sample. The sample could be, for example, a blood sample.

The geometry and/or structure of the microfluidic device 10 need not be as described above. Any microfluidic device capable of performing the invention as claimed may be used.

Any suitable PCR reagents may be used.

In the example described above, all of the reagents needed for the PCR reaction are included in the matrix of the PCR gel 54. However, this need not be the case. For example, some of the reagents required for the PCR reaction may be incorporated in the PCR gel 54 and others may be added at the time of use. Any reagents to be added could be added in a lysing solution or in a wash solution used to wash cells into the PCR gel 54.

## Claims

1. A method of amplifying nucleic acid, comprising:
providing a microfluidic device having a space therein, the space being filled with a gel medium, at least one reagent for carrying out a PCR reaction being supported within the matrix of the gel medium within the space;
bringing a nucleic acid containing sample into contact with the gel medium and introducing the sample into the gel medium;
performing PCR amplification of nucleic acid from the sample in the space using the at least one reagent; and
wherein the sample brought into contact with the gel medium and introduced into the gel medium comprises whole cells or cell lysate without any prior separation of the nucleic acid from other components of the cells with the optional exception that filtration of cell fragments by the matrix of the gel medium may occur.

2. A method according to claim 1, further comprising analysing nucleic acid products of the PCR amplification within the microfluidic device, wherein said analysing comprises performing electrophoretic separation of the PCR products.

3. A method according to claim 1 or claim 2, wherein the at least one reagent comprises at least one of nucleoside triphosphates, primer nucleic acid and polymerase for performing the PCR reaction.

4. A method according to any one of claims 1 to 3, wherein the gel medium has a homogenous composition.

5. A method according to any one of claims 1 to 4, wherein the at least one reagent is homogenously distributed throughout the gel medium.

6. A method according to any one of claims 1 to 5, wherein the sample is a buccal swab sample.

7. A method according to any one of claims 1 to 5, wherein the sample is a blood sample.

## Patentansprüche

1. Verfahren zur Nukleinsäureamplifikation, das Folgendes umfasst:
Bereitstellen einer mikrofluidischen Vorrichtung, die einen Raum darin aufweist, wobei der Raum mit einem Gelmedium gefüllt ist, wobei mindestens ein Reagenz zum Durchführen einer PCR-Reaktion innerhalb der Matrix des Gelmediums in dem Raum gestützt wird;
Inkontaktbringen einer nukleinsäurehaltigen Probe mit dem Gelmedium und Einführen der Probe in das Gelmedium;
Durchführen einer PCR-Amplifikation von Nukleinsäure aus der Probe in dem Raum unter Verwendung des mindestens einen Reagenzes; und
wobei die Probe, die mit dem Gelmedium in Kontakt gebracht wurde und in das Gelmedium eingeführt wurde, ganze Zellen oder Zelllysat ohne vorherige Trennung der Nukleinsäure von anderen Komponenten der Zellen mit der optionalen Ausnahme umfasst, dass eine Filtration von Zellfragmenten durch die Matrix des Gelmediums erfolgen kann.

2. Verfahren nach Anspruch 1, ferner umfassend Analysieren von Nukleinsäureprodukten der PCR-Amplifikation in der mikrofluidischen Vorrichtung, wobei das Analysieren Durchführen einer elektrophoretischen Trennung der PCR-Produkte umfasst.

3. Verfahren nach Anspruch 1 oder Anspruch 2, wobei das mindestens eine Reagenz mindestens eines von Nukleosidtriphosphaten, Primer-Nukleinsäure und Polymerase zum Durchführen der PCR-Reaktion umfasst.

4. Verfahren nach einem der Ansprüche 1 bis 3, wobei das Gelmedium eine homogene Zusammensetzung aufweist.

5. Verfahren nach einem der Ansprüche 1 bis 4, wobei das mindestens eine Reagenz homogen im Gelmedium verteilt ist.

6. Verfahren nach einem der Ansprüche 1 bis 5, wobei die Probe eine Wangenabstrichprobe ist.

7. Verfahren nach einem der Ansprüche 1 bis 5, wobei die Probe eine Blutprobe ist.

## Revendications

1. Procédé d'amplification d'un acide nucléique, comprenant :
la fourniture d'un dispositif microfluidique comportant un espace à l'intérieur, l'espace étant rempli d'un milieu gélifié, au moins un réactif pour réaliser une réaction de PCR étant supporté au sein de la matrice du milieu gélifié au sein de l'espace ;
la mise en contact d'un échantillon contenant un acide nucléique avec le milieu gélifié et l'introduction de l'échantillon dans le milieu gélifié ;
la réalisation d'une amplification par PCR de l'acide nucléique provenant de l'échantillon dans l'espace en utilisant l'au moins un réactif ; et
dans lequel l'échantillon mis en contact avec le milieu gélifié et introduit dans le milieu gélifié comprend des cellules entières ou un lysat cellulaire sans aucune séparation préalable de l'acide nucléique des autres composants des cellules à l'exception facultative qu'une filtration des fragments cellulaires par la matrice du milieu gélifié puisse se produire.

2. Procédé selon la revendication 1, comprenant en outre l'analyse des produits d'acide nucléique de l'amplification par PCR au sein du dispositif microfluidique, dans lequel ladite analyse comprend la réalisation d'une séparation électrophorétique des produits de PCR.

3. Procédé selon la revendication 1 ou la revendication 2, dans lequel l'au moins un réactif comprend au moins l'un des nucléosides triphosphates, un acide nucléique amorce et une polymérase pour effectuer la réaction de PCR.

4. Procédé selon l'une quelconque des revendications 1 à 3, dans lequel le milieu gélifié a une composition homogène.

5. Procédé selon l'une quelconque des revendications 1 à 4, dans lequel l'au moins un réactif est distribué de façon homogène dans tout le milieu gélifié.

6. Procédé selon l'une quelconque des revendications 1 à 5, dans lequel l'échantillon est un échantillon d'écouvillon buccal.

7. Procédé selon l'une quelconque des revendications 1 à 5, dans lequel l'échantillon est un échantillon de sang.
